# EUROPEAN PATENT APPLICATION

(11) **EP 3 192 551 A1**
(43) Date of publication of application: **19.07.2017**
(21) Application number: 16165072.6
(22) Date of filing: 13.04.2016
(51) Int. Cl.: A61M 16/08, A61G 12/00, A61G 7/05, A61G 13/10, A61M 5/14

(54) **DETACHABLE AND TRANSPORTABLE CPAP HOSE HOLDER**

(30) Priority: 14.01.2016 AU 2016200226
(71) Applicant: CrackNut AS, 5936 Manger (NO)
(72) Inventor: Tonning, Kenneth, 5936 Manger (NO)
(74) Representative: Acapo AS

(57) **Abstract**

The present invention relates to a CPAP hose holder device for the support of a CPAP hose. The device comprises a base, a hose entryway with one or more openings for a CPAP hose, entryway hose guides, an elongated support, support hose guides, and a platform, wherein: a bottom of the hose entryway is connected to the base, and a top of the hose entryway is connected to a bottom of the support, and the platform is connected to a top of the support; and the entryway hose guides are connected to the back of the hose entryway, and the support hose guides are attached to the back of the support; wherein: the hose guides and support are arranged to partially or fully enclose the CPAP hose; and the support is substantially upright with respect to the base, and the base and platform extend from the support in substantially the same direction. The invention also relates to use of said CPAP hose holder, a method of moving of the CPAP hose to another position in the bed while sleeping, and a CPAP hose holder system.

## Description

### THE FIELD OF THE INVENTION

The present invention relates to a CPAP hose holder device for the support of a CPAP hose and, more particularly, for the support a CPAP hose used with a CPAP machine. The present invention also relates to use of said CPAP hose holder, a method of moving of the CPAP hose to another position in the bed while sleeping, and a CPAP hose holder system. It further relates to relieving some of the inconvenience of sleep apnea treatment and associated methods, systems, and uses.

### BACKGROUND

Sleep apnea is a serious condition that causes individuals to repeatedly stop breathing as they sleep. The most common form is obstruction sleep apnea (OSA). When people afflicted with sleep apnea are asleep, the back of their throat muscles relax, and their airways close. The individuals will often wake up snorting, choking, or gasping for breath. Depending on the severity, this can occur hundreds of times in a night.

There are serious risks associated with sleep apnea. The constantly interrupted sleep reduces the amount of time the person spends in deep restful phases of sleep. Most people with sleep apnea are tired during the day time. Individuals afflicted with sleep apnea are shown to have a higher rate of high blood pressure, heart problems, diabetes, and liver problems. Additionally, sleep apnea can have a negative impact upon the quality of sleep for other people as well; with partners often sleeping in another room.

In cases where the sleep apnea is moderate to severe, the person will often be advised to use a continuous positive airway pressure (CPAP) machine. The individual wears a mask (nasal or nasal/oral), that is connected to a hose, which is connected to a CPAP machine. The machine will pump air through the hose, and to the individual, that is at a higher pressure than the surrounding air. This pressure prevents the upper airways from collapsing during sleep.

### TECHNICAL PROBLEM TO BE SOLVED

Although the CPAP system is a very effective and reliable treatment, many of those afflicted find it cumbersome and uncomfortable. When the individual is sleeping, the hose is usually placed on their chest or next to them in the bed. It is common for the hose to become stuck in the bedding or to the individual. When they roll over, or move in the bed, this can cause the hose to pull off the mask. This interrupts their sleep as the individual must reattach their mask; thereby defeating the purpose of using the CPAP system in the first place. This problem can be very frustrating and cause the person to stop using the device and thus risking their health. There have been a number of attempts to solve this problem of the hose dislodging the mask as the individual sleeps.

Some of these solutions require the attachment of permanent devices or structures to the headboard, wall, or ceiling. These solutions are not portable when the individual needs to travel. Additionally these structures can not be dissembled quickly if needed. They can also present a safety hazard if someone makes contact with them at speed. An individual may be unable or unwilling to attach things to their wall, ceiling, or headboard.

An individual can a place a stand on the floor. These devices tend to be large, unwieldly, and are difficult to move. Also, the holder's clinical appearance can create additional problem of making the individual feel like they are in a hospital. The sheer size of these devices can make them difficult to travel with.

An individual can use devices that are wedged between the mattress and box springs. These take time to disassemble, particularly if the bed is up against a wall. It is not convenient to move these devices every time someone wishes to make or play in the bed. These solutions also do not function well when an individual goes camping and no box springs are available.

There are products on the market that route the CPAP machine hose into a special pillow. The individual then attaches their mask to the pillow by means of a second hose. This requires the user to decide before they sleep which side of the pillow they will sleep upon. When the individual rolls from one side of their body to the other, the mask can either be pulled off or the hose can lay uncomfortably across the users face. Also, the individual has no choice but to take the special pillow with them when traveling.

A further problem with these solutions is how the hose is attached to the holder itself. These are often simply draped over the body of the holder. When the hose is attached, it is usually with only few clips between the holder and the hose. This makes the hose and holder system vulnerable when someone bumps it. This kind of treatment can be expected in many situations; for example: playing with children.

A major problem that most of these solutions have in common is that they are fixed in one position with respect to the bed. These solutions attempt to solve the problem of the individual turning from one side to the other side. But they fail to address if the individual wishes to move to a different location in the bed.

Individuals with larger beds and/or those with a partner in the same bed will move laterally on the bed as well. As the individual moves away from the point where the holder and hose connect, the chance increases of the mask dislodging. Previous solutions require that the individual decides where they are going to sleep in the bed before they go to sleep. If the individual moves to a different position in the bed, the tension between the hose holder and the mask can pull the mask off.

Therefor there is a need for a device to be used with a CPAP system that would reduce the number of times the mask is pulled off or the hose gets in the way; thereby increasing the individual's quality of sleep.

### SHORT SUMMARY OF THE INVENTION

The CPAP hose holder device in accordance with the present invention is characterized by a base, a hose entryway with one or more openings for a CPAP hose, entryway hose guides, an elongated support, support hose guides, and a platform, wherein: a bottom of the hose entryway is connected to the base, and a top of the hose entryway is connected to a bottom of the support, and the platform is connected to a top of the support; and the entryway hose guides are connected to the back of the hose entryway, and the support hose guides are attached to the back of the support; wherein: the hose guides and support are arranged to partially or fully enclose the CPAP hose; and the support is substantially upright with respect to the base, and the base and platform extend from the support in substantially the same direction.

In accordance with a preferred embodiment, the support is comprised of two or more separate parts, preferably two. The support can be curved or straight, and is preferably straight. The device may have a cover. The device is preferably arranged to be partially or wholly dissembled. A pillow can be temporarily attached to the device

Use of the CPAP hose holder device in accordance with the present invention is characterized by the prevention of face mask removal during sleep, wherein a hose from a CPAP machine is placed inside the device and a pillow is placed on the base of the device.

The method of moving of the CPAP hose device in accordance with the present invention is characterized by comprising the following steps: (a) the individual lifts their head from their pillow; (b) the individual moves the CPAP hose holder and pillow together to a new position in the bed; (c) the individual places their head back upon the pillow.

The CPAP hose holder system in accordance with the present invention is characterized by comprising: (a) a CPAP hose holder device in accordance with the present invention; (b) a pillow; (c) a CPAP machine; (d) a CPCP hose; and (e) a CPAP facemask.

### PURPOSES AND ADVANTAGES OF THE PRESENT INVENTION

The CPAP hose holder in accordance with the present invention is a device used for the support of a CPAP hose during the treatment of sleep apnea. The present invention meets the deficiencies of previous solutions to the technical problems discussed above as well as other previously unmet needs. The present invention reduces the chance that the face mask will be removed because of movement while the individual sleeps; both by rolling over or by moving to a different location in bed.

The present invention is not affixed to a wall, table, headboard, ceiling, etc. This gives the individual flexibility in where they will use the device. It also reduces the hazards of impact with this device while in the assembled position.

The present invention is not very large. It can be disassembled into several pieces and stored in a CPAP system travel bag. Additionally, the device can be broken down quickly. These factors, among others, make the device well suited for travel. These also make the device well suited for fast disassembly before making the bed or engaging in other activities where the presence of a CPAP hose or hose holder are undesirable.

The hose sits in the present invention such that it provides both protection to the hose and helps the hose stay in place. The hose is partially hidden from view which gives improves upon the aesthetics, particularly when compared to previous solutions. In addition, the holder can be decorated to fit in with the surroundings, such as cartoon prints for a child.

The present invention can be used with any pillow. This allows the individual more flexibility as they travel. There is no need to take along special pillows. The device can also be used while camping.

The present invention allows the individual to decide where they are going to sleep in the bed. It is a simple matter to move the device during sleep by moving the pillow. This allows the individual to move their sleep location at will. It does not require them to decide where they are going to sleep in the bed when they go to sleep.

### BRIEF DESCRIPTION OF THE FIGURES

FIG 1 discloses an isometric view from the front of the invention.
FIG 2A discloses an isometric view from the rear of the invention.
FIG 2B discloses an isometric view from the rear an alternate embodiment of the invention.
FIG 3 discloses a side view of the connection between the upper and lower supports.
FIG 4 discloses a top view of the base.
FIG 5 discloses a detailed front view of the lower support.
FIG 6A discloses a detailed side view of the lower support.
FIG 6B discloses a detailed side view of an alternate embodiment of the invention.
FIG 7 discloses a detailed side view of the upper support.
FIG 8A discloses a detailed rear view of the upper support.
FIG 8B discloses a detailed side view of an alternate embodiment of the upper support.
FIG 8C discloses a detailed side view of an alternate embodiment of the upper support.
FIG 9A discloses a detailed top view of the platform.
FIG 9B discloses a detailed top view of an alternate embodiment of the platform.

### DETAILED DESCRIPTION OF THE INVENTION

Using the attached drawings, the technical contents, and detailed descriptions, the present invention is described. Alternate embodiments will also be presented.

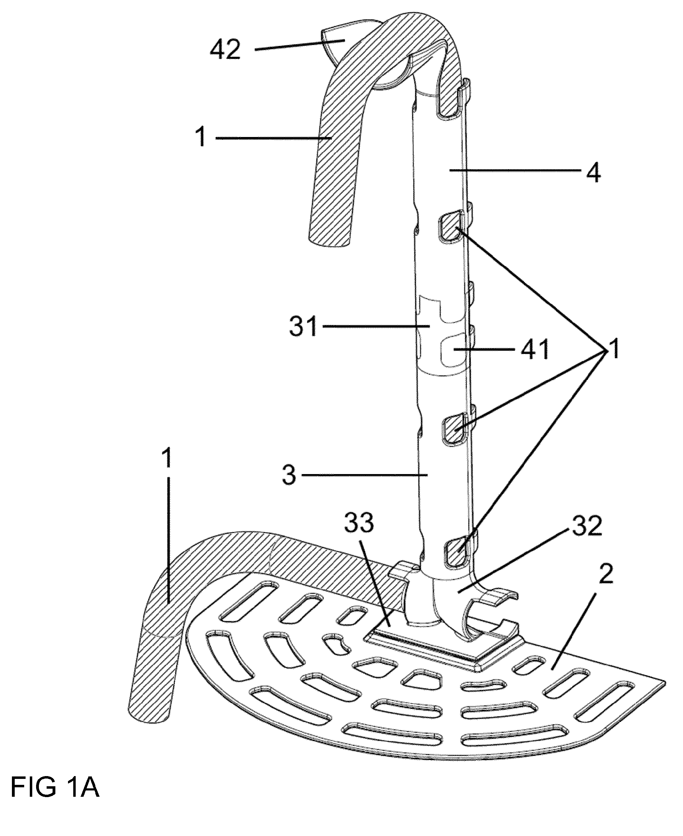

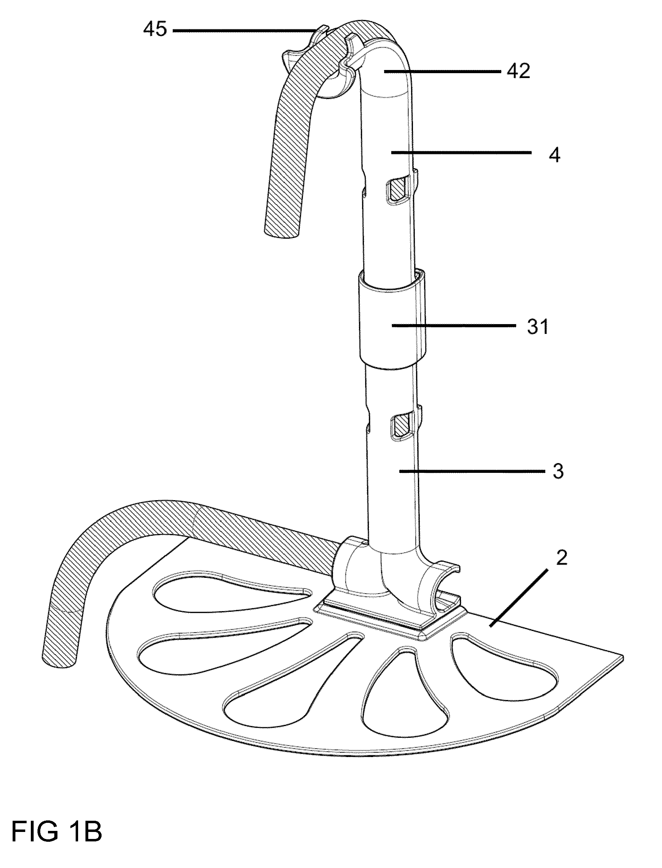

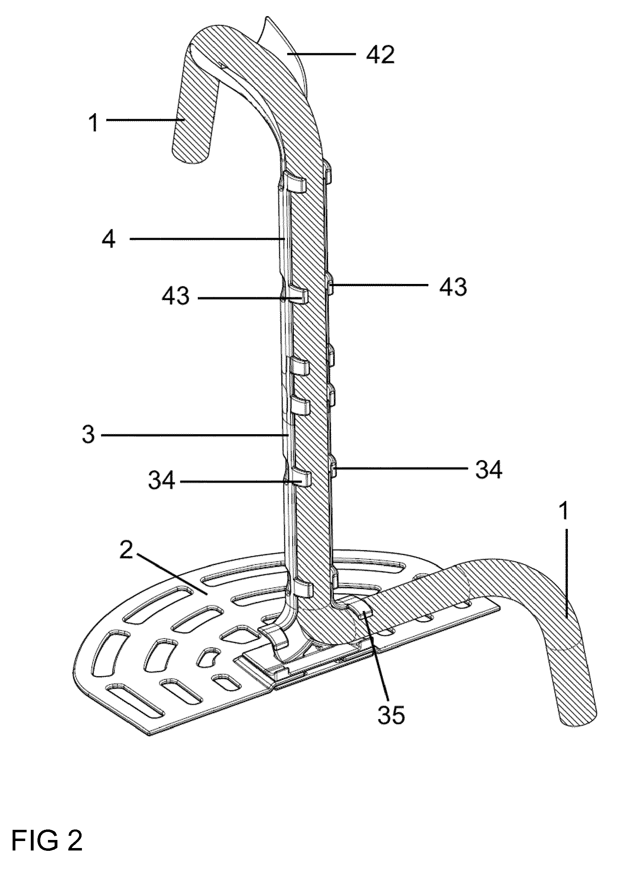

FIGS 1A, 1B, and 2 disclose isometric views of the invention. The hose 1 enters the present invention through the hose entryway 32. The hose 1 is found inside the lower support 3. This lower support 3 is connected to the base 2 by a base connection means 33. The lower support 3 is connected to the upper support 4 by the lower connection means 31 and the upper connection means 41. The hose 1 rests upon the platform 42. The hose 1 is removably held inside the invention by means of an entryway hose guides 35, lower hose guides 34, and upper hose guides 43. A pillow (not shown) is placed on top of the base 2. A CPAP face mask and CPAP machine (not shown) connects to the ends of the hose. The upper 4 and the lower 3 support combine to make a single support. The preferred embodiment is with a hose entryway 32 with two openings where the hose can enter. But it is also possible to have a hose entryway 32 with one opening or with three or more openings.

FIG 1B shows an alternate embodiment of the present invention. The hose rests upon a platform 42 that has hose guides 45. Additionally, it has a cross section profile similar to that of the top support 4. The upper connection means 41 fits entirely inside of the lower connection means 31 and is thus hidden from view. The base 2 show in FIG 2 has a different number and size of holes than the other figures.

While the upper 4 and lower 3 supports are shown with holes 36 and 43, an alternate embodiment could be without holes. FIGS 1 and 2 show the lower connection means 31 on top of the upper connection means 41, this is not the only possible configuration. The upper connection means 41 could lie on the lower connection means 31. Additionally, the connection between the upper support 4 and the lower support 3 could be achieved in a number of different ways. Examples include, but are not limited to, a treaded connection, clamps, Velcro, spring type quick connections, etc. The hose guides, 35 and 34 and 43, can be shaped different than the arcs that are depicted in the figures. Additionally they are not required to be immobile with respect to the lower support 4. Alternate embodiments of the hose guides of the present invention could include, but are not limited to: flexible, spring type, buckle, or latches. The shape of the platform 42 shown in the figures of the present invention can be different than that shown. Examples of different shapes include, but are not limited to, square, rectangle, circular, flat, curved, etc. While the back part of the present invention is shown as open, an alternate embodiment could include a cover.

Hose guides hold and guide the CPAP hose. Herein "entry hose guides" refer to hose guides affixed to the entryway, while "support hose guides" refer to hose guides connected to the support. The main objective of the hose guides is to hold and guide the CPAP hose. The main objective of the support is to form the structure that the hose and guides are connected to and supported by. Usually the hose guides only partially enclose the hose. Under normal circumstances, this is the preferred embodiment. However, in some circumstances these advantages may be outweighed by other needs. For instance, if it is desired for aesthetic reasons for the hose to be as concealed as possible, the hose guides can be constructed to fully encompass the hose. This may also be done by molding the hose guides and support as one piece, or by adding a protective sleeve to the outside of the device. Such a sleeve or fully hose enclosing structure can in accordance with a preferred embodiment be produced in an insulating material. This insulating material can for example be neoprene, Styrofoam or knitted fabrics, and would provide an advantage if the CPAP machine is to be used in low temperatures. During low temperatures, such as during the winter, it is a known problem that condensation forms in the hose. By providing insulation this problem could be alleviated.

The present invention can be made out of a large range of different materials. Examples include, but are not limited to: wood, metal, paper, plastic, or combinations thereof. In hospital use, it could be preferable for the current invention to be made out of materials that can be sterilized; such as metal. Additionally, each piece of the present invention could be made out of the same or different material.

The figures show support is shown as two different pieces: the lower support 3 and the upper support 4. An alternative embodiment can be a support made of a single piece, or of more than two pieces.

Although the figures show the support as being made up of two support pieces; a lower and upper support; this is a preferred embodiment but it is not the only embodiment of the present invention. This embodiment avoids having one long support piece when disassembling and travelling with the device, and makes it easier to pack. Other embodiments are to construct the support as one long piece, thereby making both the construction and assembly easier. The support could also be made of three or more pieces; allowing for more customization of the total support length. While a straight support is the preferred embodiment, it can also be curved if needed. This would be useful in situations where the individual has very little space above their pillow.

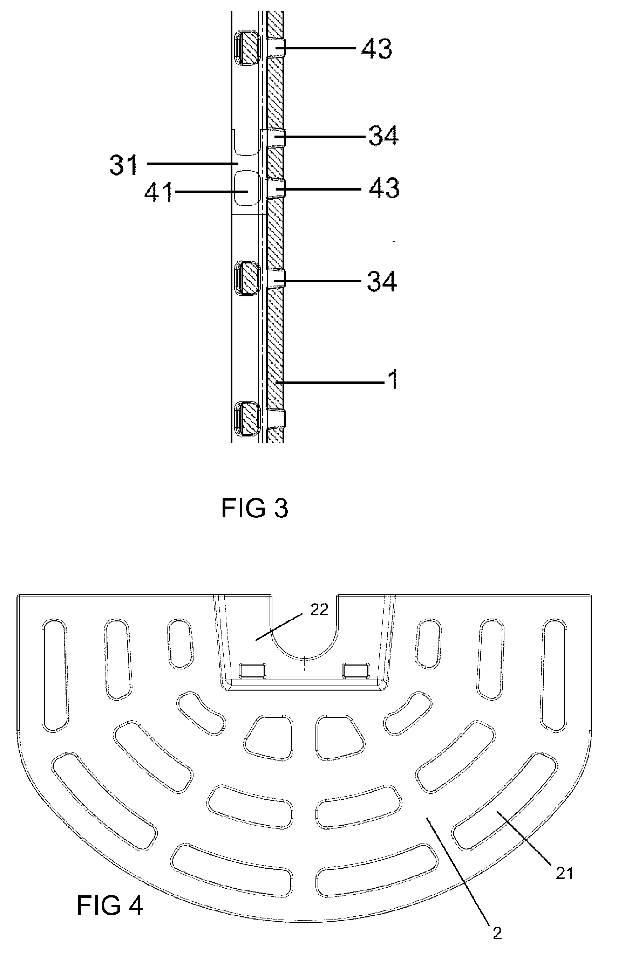

FIG 3 discloses a side view of the connection between the upper 4 and lower 3 supports of the present invention. The hose 1 is held in place by the lower hose guides 34 and the upper hose guides 43. The upper 4 and lower 3 supports are connected by means of the upper connection means 41 and the lower connection means 31.

The figures show hose guides, 34 and 43, as single molded pieces on the upper 4 and lower 3 supports. However, alternative embodiment could include hose guides that are more than a single piece. An alternate embodiment of the present invention could use straps, springs, clamps, or other mechanical means for in the place of, or in addition to, the shown hose guides. In an alternate embodiment, the hose guides could be removable from the support.

The connection between the upper 4 and lower 3 supports of the present invention is shown in FIG 3 as the upper support 4 connection means sliding into the lower support 3. However, there are other alternatives. The lower support could slide into the upper support. Other possible solutions include, but are not limited to, screwing any support pieces together, a clamping mechanism between the support pieces, a support telescoping from another support, and a hinge between the support pieces. The support pieces can be connected by one or more different connection means.

FIG 4 discloses a top view of the base 2. The base 2 contains base holes 21. The base 2 is connected to the lower support 3 at the base connection section 22.

A pillow (not shown) can be placed upon the base 2. FIG 4 shows a base plate with holes 21. Alternative embodiments could include a base with no holes. The holes make the base lighter, and allows for circulation. Additionally the base could have texturing; improving its grip to the bed and/or pillow and reducing undesired sliding. The pillow could be attached, temporarily or permanently, to the present invention by an attachment means or by base texturing. The attachment means could be connected to the present invention at the base or other parts of the device. Additionally, a lip or protrusion could be placed at the back of the present invention so it is partially held in place by the edge of the mattress.

In the present invention, the base connection means 33 is slid onto the base connection section 22. There are several alternatives including, but not limited to, hinges, pins, threaded connections, and/or clamps. It is also possible that the connection between the connection means 33 and the connection section 22 be adjustable. This could be a type connection that allows the hose entryway 32 to lean backward and forward and/or side to side with respect to the plane of the base.

Similar types of adjustment mechanisms could also be implemented at the connections between other parts of the present invention. These kinds of connections could be necessary if a high degree of positional adjustment was desired.

The present invention can be used with any pillow. There are cases, however, where the base size will need to be changed. An example of this would be a small pillow made for a child. The base is shown in FIG 4 as a rounded rectangle, but a circular, square, rectangular, or other base shapes would be acceptable. The size and shape of the pillow and the size and shape of the base 2 should therefore be somewhat adjusted to each other. Preferably, the base should not be so large as to protrude beyond the pillow, as this might be uncomfortable, unsightly, and would expose the base to unintended movement. However, the base also should be large enough to form a solid base in order for the support to stay upright during use. When in use, both the pillow and the head of the individual resting on the pillow aids in this, as their weight and friction will keep the base oriented correctly, i.e. flat on the bed so that the supports 3 and 4 are kept vertical. The base should therefore be large enough for a head resting on the pillow to hold it in place.

While FIG 4 shows that the base connection section 22 is centered on the rear part of the base 2, this choice of location is not the only choice. There are foreseeable situations where this connection will need to be made at a different place; for example: to work around the position of other equipment in the bed. The base connection section 22 could be found anywhere on the baseplate, but preferably toward the rear of the base, most preferably centered toward the rear of the base.

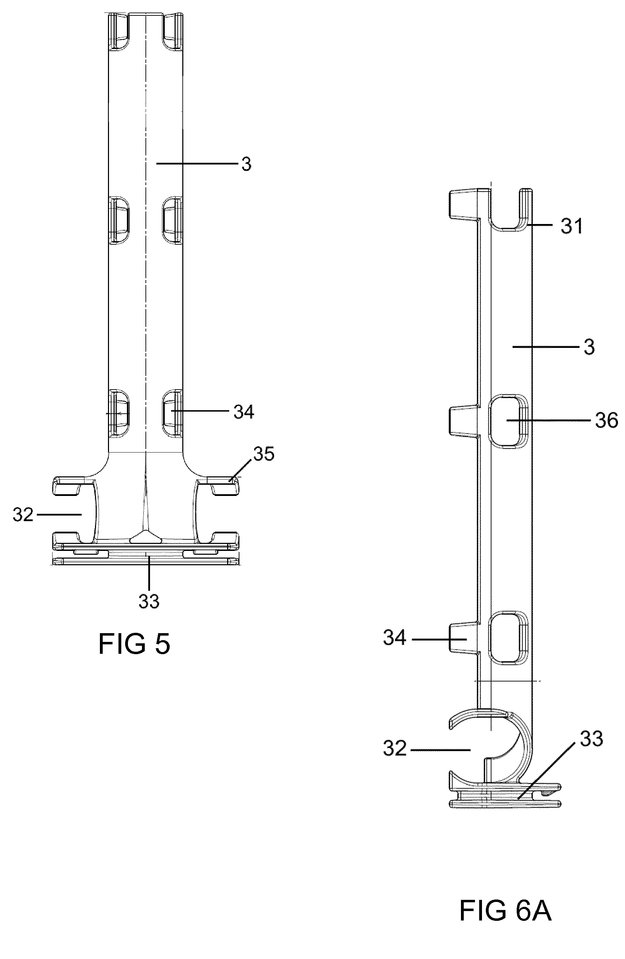

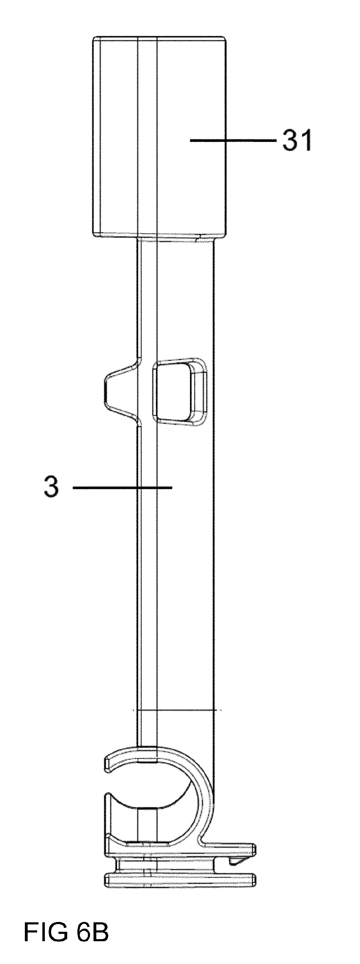

FIGS 5 and 6 disclose detailed views of the lower support 3. The entryway 32 is connected to an entryway hose guide 35. A base connection means 33 is connected to the bottom of the lower support 3. Lower hose guides 34 are also shown. There are holes 36 in the lower support 3. The top of the lower support 3 contains the lower connection means 31.

FIG 6B disclose a detailed view of an alternative embodiment of the present invention with a different lower connection means than that shown in FIG 6A.

FIGS 5, 6A, and 6B show a relatively small distance between the two different hose entryways 32, alternative embodiments could have distance that are larger or smaller than shown.

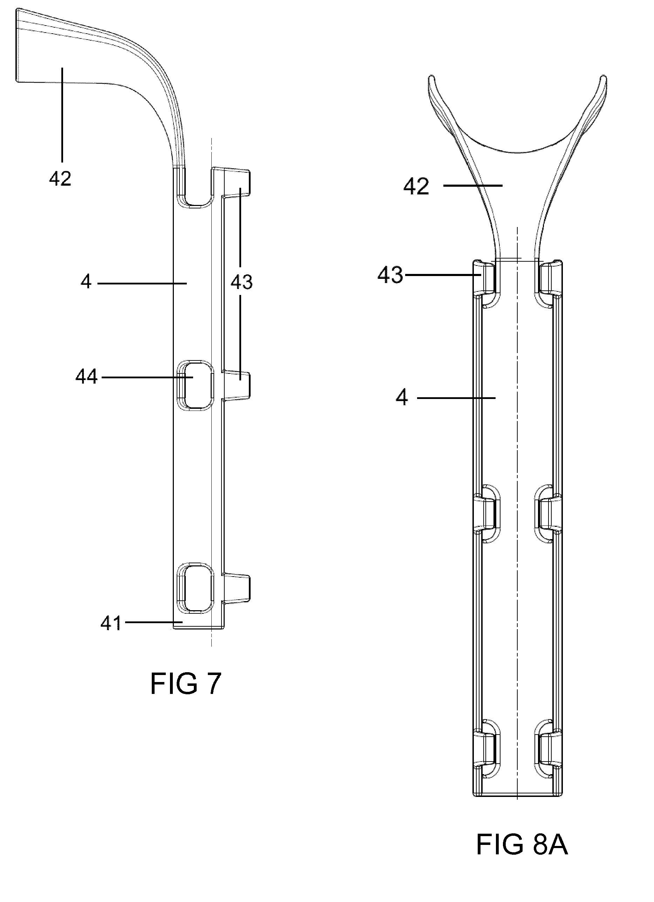

FIG 7 and 8A disclose detailed views of the upper support 4. The upper connection means 41 is found on the bottom part of the upper support 4. Holes 44 are found in the upper support. The upper hose guides 43 are connected to the upper support 3. The platform 42 is connected to the top part of the upper support.

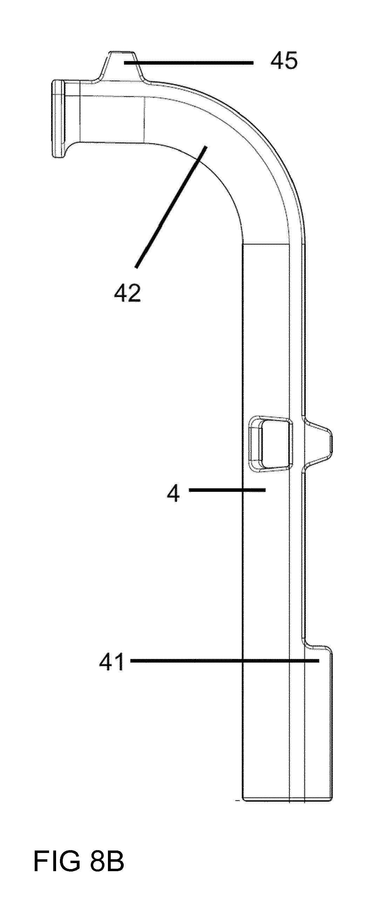

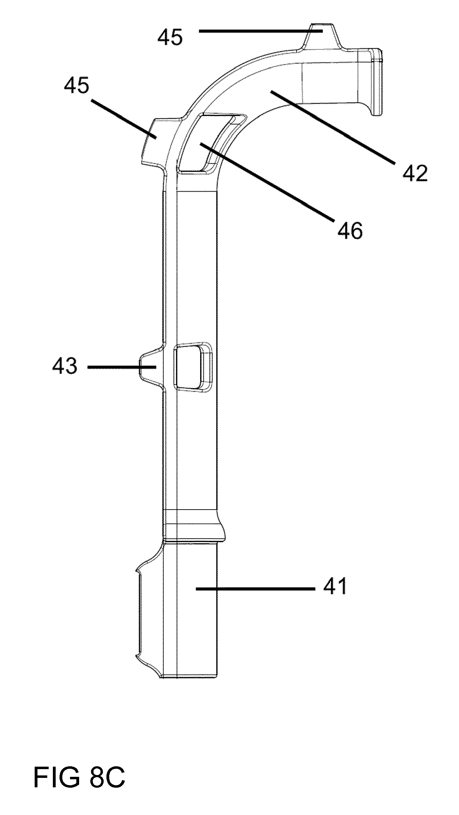

FIG 8B discloses a side view of an alternative embodiment of the upper support 4. It uses a different type of upper connection means 41. It also shows another possible shape for the platform 42 and the addition of a platform hose guide 45. FIG 8C also discloses a side view of an alternative embodiment of the invention. In this embodiment, the platform 42 has a second platform hose guide 45. Also, the platform 42 has holes 46 and another embodiment of the upper connection means 41.

While the figures show the platform as a flat surface, it an alternative would be a platform with a clamp or slot for the CPAP hose.

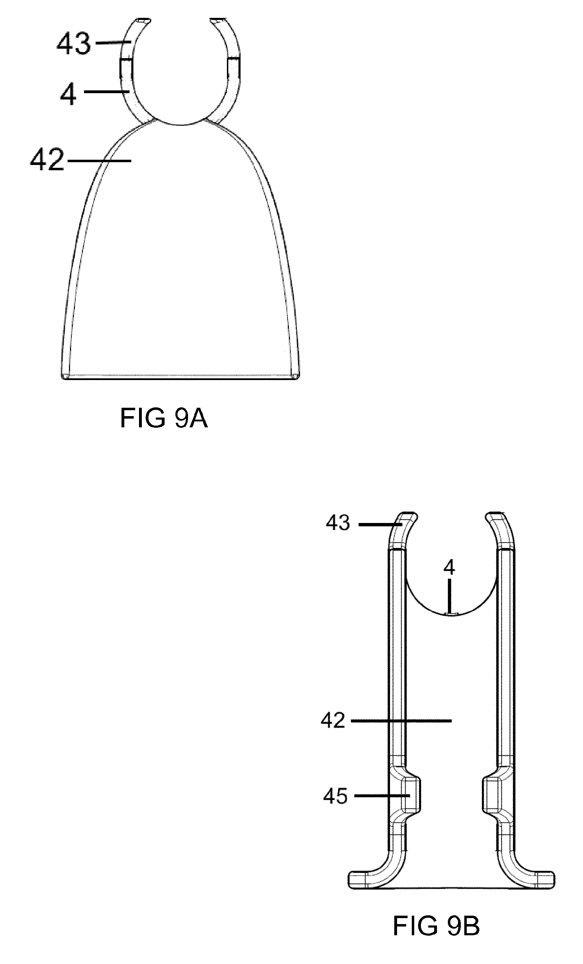

FIG 9A discloses a detailed top view of the platform 42. The platform 42 is connected to the upper support 4. The upper hose guide 43 is connected to the upper support 4.

FIG 9B discloses a top view of an alternative embodiment of the invention. In this embodiment, the platform 42 has the same width as the upper support 4. The platform hose guides 45 are connected to the platform 42.

While the figures show that the platform 42 and the upper support 4 as a single molded piece, these could be two separate pieces.

Use of the CPAP hose holder for the prevention of face mask removal during sleep shall now be explained. The CPAP machine is connected to the face mask by a hose. The individual places the base 2 of the present invention directly under the individual's pillow. Preferably the base 2 is placed under the pillow so that the support 3 protrudes upward from a position above the individuals head, although it could also be protruding from the left or right side of the individuals head. This hose 1 is connected to the CPAP machine and placed in the present invention. The hose 1 exits the upper platform 42 and hangs freely down towards the individuals face. At the end of the hose the facemask is connected. The individual sleeps with the facemask on, as normal. When the individual turns his head or otherwise moves his face, the hose, due to being free hanging above the individual, will not become tangled up, and be in the way or pulled upon, thus preventing face mask removal.

The method for the moving of the hose holder, while the CPAP hose is attached, during sleep is given by the following steps:
a) The individual lifts their head.
b) The individual moves the hose holder and pillow together to the desired location.
c) The individual places their head back upon their pillow.

Since the base 2 is flat and fitted under the pillow, it is easy to move with the pillow even if it is not attached thereto. Sliding the pillow around in bed while mostly asleep is therefore possible, greatly improving the ability of CPAP users to change position in bed and therefore sleep more naturally.

Even though the CPAP hose holder is described herein as a separate system, it is meant to be able to function as a part of a system that is comprised of:
a) The present invention.
b) A pillow.
c) A CPAP machine.
d) A CPAP hose.
e) A CPAP face mask.

The CPAP machine is connected to the CPAP face mask by means of a CPAP hose. This hose is placed inside the present invention. The pillow is then placed on top of the CPAP hose holder.

The present invention is made to fit under a pillow. The size of the base and the total height of the support can be changed as needed to fit any pillow. There is no international standard pillow size. Many manufactures simply do not adhere to any dimensions. Table 1 gives the most common standard sizes for Europe and North America.

**Table 1 - standard pillow sizes for Europe and North America**

| | Depth (cm) | Width (cm) |
|---|---|---|
| Toddler & Travel | 41 | 51 |
| Regular | 51 | 66 |
| Standard | 51 | 71 |
| Queen | 51 | 76 |
| King | 51 | 91 |
| European | 66 | 66 |

These large variations in pillow sizes account for the variations in the dimensions of the preferred embodiment.

The present invention is designed to fit a hose of a known diameter. Presently the standard for CPAP hoses is a 22mm outer diameter. Thus supports for the present standard CPAP hoses should have an inner diameter of 22mm. If this changes, the diameter of the supports of the present invention could be changed to fit this new size.

The width of the base 2 can be 15 to 40 cm, preferably 25 to 35 cm, most preferably 30 cm. The depth of the base 2 can be 10 to 30 cm, preferably 15 to 25 cm, most preferably 20 cm.

The height of the lower support 3 together with the hose entryway 32 and lower connection means 31 can be 10 to 35 cm, preferably 15 to 30 cm, most preferably 22.6 cm. The distance between the two hose entryway 32 of the lower support 3 can be 4 to 35 cm, preferably 6 to 20 cm, most preferably 7.6 cm.

The height of the upper support 4 together with the platform 42 and the upper connection means 41 can be 10 to 30 cm, preferably 15 to 25 cm, most preferably 18 cm. The width of the upper support can be 2 to 6 cm, preferably 2.5 to 4 cm, most preferably 3 cm.

The height of the platform 42 of the upper support 4 can be 3 to 10 cm, preferably 4 to 8 cm, most preferably 6.5 cm. The width of the platform 42 of the upper support 4 can be 2 to 15 cm, preferably 4 to 10 cm, most preferably 5 cm. The platform 42 overhangs the top support 4 by between 1 and 15 cm, preferably 9.2 cm.

The total height of the present invention, from bottom of base 2 to the top of the platform 42, can be 20 to 60 cm, preferably 30 to 50 cm, most preferably 42 cm. The interior diameter of the supports of the present invention can be 15 to 30 mm, preferably 20 to 26 mm, most preferably 22 mm.

### LIST OF USED REFERENCE NUMERALS AND ACRONYMS:

- 1: CPAP Hose
- 2: Base
- 21: Base Hole
- 22: Base Connection Section
- 3: Lower Support
- 31: Lower Connection Means
- 32: Hose Entryway
- 33: Base Connection Means
- 34: Lower Hose Guide
- 35: Entryway Hose Guide
- 36: Lower Support Hole
- 4: Upper Support
- 41: Upper Connection Means
- 42: Platform
- 43: Upper Hose Guides
- 44: Upper Support Hole
- 45: Platform Hose Guide
- 46: Platform Hole

## Claims

1. A CPAP hose holder device for the support of a CPAP hose comprising:
a base, a hose entryway with one or more openings for a CPAP hose, entryway hose guides, an elongated support, support hose guides, and a platform, wherein:
a bottom of the hose entryway is connected to the base, and a top of the hose entryway is connected to a bottom of the support, and the platform is connected to a top of the support; and
the entryway hose guides are connected to the back of the hose entryway, and the support hose guides are attached to the back of the support; wherein:
the hose guides and support are arranged to partially or fully enclose the CPAP hose; and
the support is substantially upright with respect to the base, and the base and platform extend from the support in substantially the same direction.

2. A CPAP hose holder device in accordance with claim 1, wherein the support is comprised of two or more separate parts, preferably two.

3. A CPAP hose holder device in accordance with claim 1 or 2, wherein the support can be curved or straight, preferably straight.

4. A CPAP hose holder device in accordance with claim 1 or 2, wherein the device has a cover.

5. A CPAP hose holder device in accordance with any of claims 1-3, wherein the device is arranged to be partially or wholly dissembled.

6. A CPAP hose holder device in accordance with any of claims 1-4, wherein a pillow can be temporarily attached to the device.

7. Use of a CPAP hose holder device in accordance with any of the previous claims for the prevention of face mask removal during sleep, wherein a hose from a CPAP machine is placed inside the device and a pillow is placed on the base of the device.

8. Method of moving of the CPAP hose holder device in accordance with claim 1 to another position in the bed while sleeping, comprising the following steps:
a. the individual lifts their head from their pillow;
b. the individual moves the CPAP hose holder device and pillow together to a new position in the bed;
c. the individual places their head back upon the pillow.

9. A CPAP hose holder system comprised of:
a. a CPAP hose holder device in accordance with claim 1;
b. a pillow;
c. a CPAP machine;
d. a CPAP hose; and
e. a CPAP facemask.
